(19) Europäisches Patentamt
European Patent Office
Office européen des brevets

(11) Veröffentlichungsnummer: **0 309 839 B1**

(12) **EUROPÄISCHE PATENTSCHRIFT**

(45) Veröffentlichungstag der Patentschrift: **04.03.92**

(21) Anmeldenummer: **88115262.3**

(22) Anmeldetag: **17.09.88**

(51) Int. Cl.$^5$: **C07C 323/19**, C07C 323/22, C07C 315/02, C07C 317/24, C07C 317/22

# (54) Verfahren zur Herstellung von Oxethylmercapto-benzaldehyden und deren Oxidationsprodukten.

(30) Priorität: **22.09.87 DE 3731763**

(43) Veröffentlichungstag der Anmeldung:
**05.04.89 Patentblatt 89/14**

(45) Bekanntmachung des Hinweises auf die Patenterteilung:
**04.03.92 Patentblatt 92/10**

(84) Benannte Vertragsstaaten:
**CH DE FR GB IT LI**

(56) Entgegenhaltungen:

**HELVETICA CHIMICA ACTA, Band 67, 1984, Seiten 1316-1327; R. SCHWYZER et al.: "The CAMET and CASET links for the synthesis of protected oligopeptides and oligodeoxynucleotides on solid and soluble supports"**

(73) Patentinhaber: **HOECHST AKTIENGESELLSCHAFT**
**Postfach 80 03 20**
**W-6230 Frankfurt am Main 80(DE)**

(72) Erfinder: **Papenfuhs, Theodor, Dr.**
**Heinrich-Bleicher-Strasse 40**
**W-6000 Frankfurt am Main 50(DE)**

EP 0 309 839 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein wirtschaftlich und ökologisch vorteilhaftes Verfahren zur Herstellung von Oxethylmercapto-benzaldehyden und deren Oxidationsprodukten (Oxethylsulfonyl-benzaldehyden und Oxethylsulfonyl-benzoesäuren) in hohen Ausbeuten und guten Qualitäten.

Die genannten Verbindungen stellen technisch wichtige Vorprodukte dar. So dient beispielsweise die 4-Oxethylsulfonylbenzoesäure als "solid phase link" zum Aufbau spezieller Oligonucleotide (Tetrahedron Lett. 25, 3967-70 (1984); Helv. Chim. Acta 1984, 1316-27). Oxethylsulfonyl-benzoesäuren sind außerdem wichtige Vorproduke zur Herstellung faserreaktiver Azofarbstoffe (US-PS 3 098 096). 2- und 4-Oxethylmercaptobenzaldehyde und ihre Oxidationsprodukte sind wertvolle Zwischenprodukte zur Herstellung von optischen Aufhellern der 1,4-Distyrylbenzolreihe (DE-OS 2 401 665) sowie zur Herstellung von Pharmazeutika (EP-OS 0 089 154).

Zur Herstellung der genannten Verbindungen sind bereits spezielle Verfahren bekannt:

2- und 4-Oxethylmercapto-benzaldehyd werden durch Umsetzung von 2- bzw. 4-Chlorbenzaldehyd mit Mercaptoethanol/KOH oder Natriummethylat erhalten. Als Lösungsmittel wird Dimethylsulfoxid/Tetrachlorkohlenstoff verwendet (DE-OS 2 401 665, Seiten 37-40, Tabelle IV). In der EP-OS 0 089 154 wird die entsprechende Umsetzung von 4-Fluorbenzaldehyd mit Mercaptoethanol beschrieben (Seiten 59/47). - Bei einer allgemeinen Methode zur Herstellung von Alkylmercapto-benzaldehyden aus Brombenzaldehyden und Alkylmercaptanen werden Dimethylformamid als Lösungsmittel und Natriumhydrid (in Mineralöl dispergiert) als Base (Synth. Commun. 16, 565-70 (1986)) verwendet. - Oxethylsulfonylbenzaldehyde werden zwar in der DE-OS 2 401 665 als Zwischenprodukt erwähnt, über ihre Synthese findet sich dort jedoch kein Hinweis. 4-Oxyethylsulfonyl-benzoesäuren werden bisher ausschließlich aus Vorprodukten hergestellt, die bereits die Carboxylgruppe enthalten. So entsteht 4-Oxethylmercapto-benzoesäure ("CAMET") durch Umsetzung von 4-Mercaptobenzoesäure mit Chlorethanol (US-PS 3 098 096) oder Ethylenoxid (Helv. Chim. Acta 1984, 1316-27). Deren Oxidation zur 4-Oxethylsulfonyl-benzoesäure ("CASET (2)") wird mit $H_2O_2$ unter Schwermetallkatalyse (Helv. Chim. Acta 1984, 1316-27) oder mit Hypochlorit (US-PS 3 098 096) beschrieben. Die letztgenannte Verbindung kann auch auf dem klassischen Weg aus 4-Chlorsulfonyl-benzoesäure (Reduktion zur Sulfinsäure und deren Umsetzung mit Ethylenoxid oder Chlorethanol) hergestellt werden (US-PS 3 098 096).

Die vorstehend erwähnten bekannten Verfahren sind ausnahmslos nur zur Herstellung spezieller Zwischenprodukte der weiter unten angegebenen allgemeinen Formel (I) geeignet und haben darüberhinaus folgende schwerwiegende Nachteile:

Bei den Austauschreaktionen an den Halogenbenzaldehyden werden teure dipolar-aprotische Lösungsmittel (Dimethylformamid oder -sulfoxid) und meist zusätzlich schwer handhabbare Basen (Alkalialkoholate oder -hydride) verwendet. Außerdem werden bei diesen Verfahren nur mäßige Ausbeuten und Reinheiten erzielt, so daß in der Regel ein Reinigungsschritt (Destillation, Extraktion mittels Chloraliphaten oder Umkristallisation aus Aromaten, beispielsweise Benzol) angeschlossen werden muß. Lösungsmittelkreisläufe sind unabdingbar.

Die Oxethylierungsreaktionen zur Bildung der Oxethylmercapto- bzw. Oxethylsulfonyl-benzoesäuren benötigen nur schwierig und daher unwirtschaftlich zugängliche Ausgangsverbindungen (Mercaptobenzoesäuren, 4-Chlorsulfonyl-benzoesäure). Aufgrund der zwangsweise anfallenden Nebenprodukte (Salze, Chlorethanol, niedrigwertige Schwefelverbindungen) sind diesen Oxethylierungsreaktionen aus Ökologiegründen aufwendige Reinigungsverfahren für Produkt und Mutterlauge nachzuschalten.

Die Oxidationsreaktionen zur Herstellung der Oxethylsulfonylbenzoesäuren verlangen teilweise ökologisch ungünstige Reagentien (wie Natriumhypochlorit) und sind bisher für die Umsetzung von entsprechenden Benzaldehyden nicht beschrieben.

Es wurde nun überraschenderweise gefunden, daß man Oxethylmercapto-benzaldehyde und deren Oxidationsprodukte Oxethylsulfonyl-benzaldehyde und Oxethylsulfonyl-benzoesäuren der allgemeinen Formel (I)

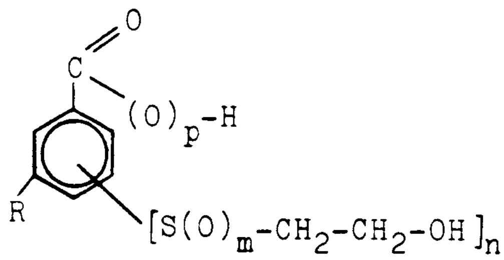

(I)

in welcher R ein Wasserstoff-, Fluor-, Chlor-, Brom- oder Jodatom, m die Zahl 0 oder 2, n die Zahl 1 oder 2, und p die Zahl 0 oder 1 bedeuten, mit der Maßgabe, daß p = 0, wenn m = 0 ist, und die Seitenkette -S-$(O)_m$-$CH_2$-$CH_2$-OH in ortho- und/oder para-Position zur Aldehyd- bzw. Carboxygruppe steht, auf wirtschaftlich und ökologisch vorteilhafte Weise in hohen Ausbeuten und guten Qualitäten herstellen kann, indem man 1 Mol eines Halogenbenzaldehyds der allgemeinen Formel (II)

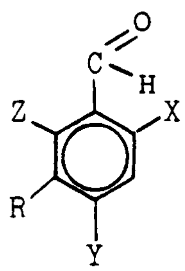

(II)

in welcher R, X, Y und Z Wasserstoff-, Fluor-, Chlor-, Brom- oder Jodatome darstellen, mit der Maßgabe, daß R, X, Y und Z in summa 1, 2 oder 3 Halogenatome sein können, wobei R ein Halogenatom ist, wenn R, X, Y und Z zusammen 3 Halogenatome darstellen, in wäßrigem Medium (in Abwesenheit organischer Lösungsmittel) mit 1,0 bis 1,5 Mol, vorzugsweise 1,25 bis 1,4 Mol Mercaptoethanol (pro auszutauschendem Halogenatom) in Gegenwart säurebindender Mittel bei Temperaturen von 70 °C bis 150 °C, vorzugsweise 90 °C bis 120 °C, zu den entsprechenden Oxethylmercapto-benzaldehyden kondensiert und diese (gegebenenfalls im Gemisch mit geringen Mengen der entsprechenden Cannizzaro-Produkte Oxethylmercapto-benzylalkohol und -benzoesäure), gegebenenfalls nach erforderlicher Zwischenisolierung, zu den entsprechenden Oxethylsulfonyl-benzaldehyden bzw. Oxethylsulfonyl-benzoesäuren mindestens mit der jeweils erforderlichen Menge Wasserstoffperoxid bei Temperaturen von 40 °C bis 110 °C, vorzugsweise 60 °C bis 95 °C, bei (zum Erhalt des entsprechenden Oxethylsulfonyl-benzaldehyds) pH-Werten, < 5, vorzugsweise pH 1-4, bzw. bei (zum Erhalt der entsprechenden Oxethylsulfonyl-benzoesäuren) pH-Werten >8, vorzugsweise 9-13, jeweils in Gegenwart einer Wolfram(VI)-Verbindung als Katalysator oxidiert.

Als Ausgangsverbindungen seien beispielsweise 2-Chlorbenzaldehyd, 4-Chlorbenzaldehyd, 2,4-Dichlorbenzaldehyd, 2,5-Dichlorbenzaldehyd, 2,6-Dichlorbenzaldehyd, 3,4-Dichlorbenzaldehyd, 2,3-Dichlorbenzaldehyd und 2,4,5-Trichlorbenzaldehyd sowie die entsprechenden Fluor- oder Bromderivate genannt.

Als säurebindende Mittel kommen Alkalimetall- und Erdalkalimetallhydroxide, -oxide, und -carbonate in Frage, wobei die Alkalimetallhydroxide und Erdalkalimetalloxide bevorzugt sind. An einzelnen säurebindenden Mitteln seien Ätzkali bzw. Kalilauge, Ätznatron bzw. Natronlauge und Magnesiumoxid genannt.

Die Kondensation wird im einzelnen in der Weise durchgeführt, daß man eine wäßrige Emulsion oder Suspension des Halogenbenzaldehyds nacheinander mit der 1,0 bis 1,5-fachen Molmenge, vorzugsweise 1,25 bis 1,4-fachen Molmenge Mercaptoethanol (pro auszutauschendem Halogenatom) und der dieser Menge äquivalenten Menge des Säurebinders versetzt, die Mischung 6 bis 20 Stunden, vorzugsweise 8 bis 15 Stunden, bei 70 bis 150 °C, vorzugsweise 90 bis 120 °C, verrührt, gegebenenfalls nicht umgesetzten Halogenbenzaldehyd mit Wasserdampf aus dem Reaktionsgemisch abdestilliert und den gebildeten Oxethylmercapto-benzaldehyd der genannten Formel (I), gegebenenfalls nach vorherigem Abkühlen auf Temperaturen von 0 bis 50 °C, vorzugsweise 10 bis 25 °C, durch Filtration oder Phasentrennung isoliert.

Je nach Reaktionstemperatur ist ein Arbeiten unter Normaldruck oder in geschlossenem System angebracht.

Die Oxidation der so erhältlichen Oxethylmercapto-benzaldehyde führt pH-wertabhängig zu definierten Oxidationsprodukten der genannten allgemeinen Formel (I): So wird bei pH-Werten unter 5, vorzugsweise

bei pH 1 bis 4 mit 2 Mol $H_2O_2$ pro Oxethylmercaptogruppe der entsprechende Oxethylsulfonyl-benzaldehyd erhalten, sofern man die Oxidation in an sich bekannter Weise mit Wolfram(VI)-Verbindungen (beispielsweise $Na_2WO_4$, $WO_3$) katalysiert.

Bei pH-Werten über 8 (vorzugsweise 9 bis 13) dagegen wird auch die Aldehyd-Funktion zur Carboxylfunktion oxidiert, so daß, in nicht näher zu spezifizierender Reihenfolge, als Endprodukt die entsprechende Oxethylsulfonyl-benzoesäure resultiert (die Zwischenverbindungen Oxethylmercapto- und Oxethylsulfinylbenzoesäure sind nicht in definierter Form faßbar). Zur vollständigen Überführung des Mono-Oxethylmercapto-benzaldehyds in die Mono-Oxethylsulfonyl-benzoesäure sind mindestens 3 Mol Wasserstoffperoxid erforderlich; enthält die Ausgangsverbindung 2 Oxethylmercaptogruppen, erhöht sich der $H_2O_2$-Bedarf auf mindestens 5 Mol. In der Praxis wird vorteilhaft ein $H_2O_2$-Überschuß von 10 bis 60 %, vorzugsweise 20 bis 35%, angewandt, um die Oxidation in vertretbaren Reaktionszeiten zu Ende zu führen und der unvermeidbaren teilweisen Selbstzersetzung des Wasserstoffperoxides Rechnung zu tragen.

Die Oxidation wird im einzelnen in der Weise durchgeführt, daß man den entsprechenden Oxethylmercapto-benzaldehyd in Wasser suspendiert oder, gegebenenfalls in Gegenwart eines Emulgators, emulgiert, mit Mineralsäure oder Alkalimetalllauge den gewünschten pH-Wert (pH 1 bis 4 zur alleinigen Oxidation der Oxethylmercaptogruppe, pH 9 bis 13 zur Herstellung der Oxethylsulfonyl-benzoesäure) einstellt, als Oxidationskatalysator eine Verbindung des 6-wertigen Wolframs (Natriumwolframat oder $WO_3$) in geringen Mengen (0,1 bis 10 g, vorzugsweise 1 bis 5 g pro Mol Mercaptoverbindung) zusetzt, nach Aufheizen auf 40 °C bis 110 °C, vorzugsweise 60 °C bis 95 °C, innerhalb 15 bis 120 Minuten die erforderliche Menge wäßriges, vorzugsweise 25 bis 40%iges Wasserstoffperoxid zutropft und bis zur Beendigung der Umsetzung (Prüfung durch beispielsweise high performance liquid chromatography ("HPLC") oder Dünnschichtchromatogramm ("DC") bei Reaktionstemperatur nachrührt. Die gebildete Zielverbindung kann dann, ggf. nach Abkühlen auf Temperaturen von 0 °C bis 50 °C, vorzugsweise 10 °C bis 25 °C, durch Filtration oder Extraktion isoliert werden.

Als besonders vorteilhaft hat sich in vielen Fällen ein Eintopfverfahren erwiesen, bei dem der in erster Stufe gebildete Oxethylmercaptobenzaldehyd nicht isoliert wird, sondern direkt in der Reaktionsmischung nach Einstellen des erforderlichen pH-Wertes und Zugabe eines Wolfram(VI)-Katalysators mittels Wasserstoffperoxid in erfindungsgemäßer Weise zur gewünschten Zielverbindung oxidiert wird.

Die nachfolgenden Beispiele sollen die Erfindung näher erläutern, ohne sie darauf einzuschränken.

Die angegebenen Gewichtsteile verhalten sich zu den genannten Volumenteilen wie g zu ml (bzw. kg zu l).

**Beispiel 1**

Zu einer Mischung aus 150 Teilen Wasser, 63,4 Teilen Ätzkali (85 %ig) und 88,3 Teilen Mercaptoethanol tropft man bei 40 °C unter gutem Rühren innerhalb 15 Minuten 114,8 Teile geschmolzenen 4-Chlorbenzaldehyd, heizt anschließend auf 90 bis 92 °C und rührt ca. 18 Stunden bei dieser Temperatur nach. Der Fortgang der Reaktion wird dünnschichtchromatografisch verfolgt. Wenn kein Ausgangsprodukt mehr nachweisbar ist, setzt man zum Lösen der ausgefallenen anorganischen Salze 320 Teile Wasser zu, kühlt unter Rühren auf 10 bis 20 °C ab und saugt den während des Kühlens in Granalien erstarrten 4-Oxethylmercapto-benzaldehyd ab. Nach Waschen mit 200 Teilen Eiswasser und Trocknen im Vakuum bei 40 °C erhält man 137,6 Teile 4-Oxethylmercapto-benzaldehyd vom Schmelzpunkt 58 bis 60 °C und einem Reingehalt von 96,9 % (HPLC). Das Produkt enthält ca. 2 % 4-Oxethylmercapto-benzylalkohol (aus der parallel ablaufenden Cannizzaro-Reaktion), der unter mäßigem Ausbeuteverlust aufgrund seiner Wasserlöslichkeit durch einmaliges Ausrühren mit 300 Teilen Eiswasser entfernt werden kann. Gewonnen werden an 4-Oxethylmercapto-benzaldehyd vom Schmelzpunkt 60 bis 61 °C und einem Reingehalt von > 99 % 126 Teile.

Aus der Mutterlauge der Umsetzung lassen sich durch Ansäuern, Abfiltrieren und Trocknen 4,5 Teile 4-Oxethylmercapto-benzoesäure vom Schmelzpunkt 149 bis 152 °C (entstanden als korrespondierendes Cannizzaro-Produkt) isolieren.

Verwendet man an Stelle des Ätzkalis eine äquivalente Menge Magnesiumoxid und arbeitet im übrigen in der angegebenen Weise, so erhält man den 4-Oxethylmercapto-benzaldehyd in vergleichbarer Ausbeute und Qualität.

**Beispiel 2**

Eine gerührte Mischung aus 250 Teilen Wasser, 195 Teilen Mercaptoethanol und 229,6 Teilen 2-Chlorbenzaldehyd wird bei 50 bis 55 °C innerhalb 30 Minuten mit 140 Teilen 85 %igem Ätzkali portionsweise versetzt. Anschließend heizt man auf 105 bis 110 °C auf (Rückfluß) und rührt ca. 15 Stunden bei dieser Temperatur nach. Dünnschichtchromatographische Verfolgung der Reaktion zeigt, daß nach dieser Zeit nur noch geringe Mengen 2-Chlorbenzaldehyd im Umsetzungsgemisch vorhanden sind. Diese werden durch indirekte Wasserdampf-Destillation (Überdestillieren der Brüden, bis das Destillat einphasig ist) entfernt. Dabei werden 6,5 Teile (2,8 % der Theorie) 2-Chlorbenzaldehyd zurückgewonnen (Phasentrennung des Destillates), die im nächsten Ansatz wieder eingesetzt werde können.

Anschließend wird die heiße Reaktionsmischung zum Lösen der anorganischen Salze mit 550 Teilen Wasser versetzt und auf 20 bis 25 °C kaltgerührt. Nach Abstellen des Rührers trennt sich das Umsetzungsgemisch in 2 Phasen. Die organische Phase wird abgetrennt, 2 mal mit entsalztem Wasser (250 bis 300 Teile) ausgerührt und durch kurzes Andestillieren im Vakuum (100 bis 150 mbar) entwässert. Man erhält 280,0 Teile 2-Oxethylmercapto-benzaldehyd vom Reingehalt 98,4 % (HPLC).

(Analyse: S: 17,5/17,7 %; ber.: 17,58 %/Restchlor: < 0,3 %; ber. 0,0 %). Ersetzt man das Ätzkali durch eine aliquote Menge 50%ige Natronlauge, die in der angegebenen Zeit gleichmäßig zugetropft wird,und arbeitet sonst in entsprechender Weise, so erhält man 10,4 Teile nicht umgesetzten 2-Chlorbenzaldehyd als Destillat und 273,8 Teile 2-Oxethylmercapto-benzaldehyd von vergleichbarer Qualität.

**Beispiel 3**

Eine Mischung aus 200 Teilen Wasser, 90,0 Teilen Mercaptoethanol und 143,0 Teilen 2,4-Dichlorbenzaldehyd wird auf 60 bis 65 °C erwärmt. Man tropft bei dieser Temperatur unter gutem Rühren innerhalb 20 Minuten gleichmäßig 110,0 Teile 50%ige Kalilauge zu und heizt anschließend auf 85 bis 90 °C.
Durch ca. 12-stündiges Nachrühren bei dieser Temperatur wird die Umsetzung vervollständigt (Kontrolle mittels HPLC auf Abwesenheit von 2,4-Dichlorbenzaldehyd.

Zum Lösen der anorganischen Salze werden 180 Teile Wasser zur heißen Reaktionsmischung zugesetzt, anschließend wird unter starkem Rühren auf 15 bis 20 °C abgekühlt, der ausgefallene 3-Chlor-4-oxethylmercapto-benzaldehyd abgesaugt, mit Wasser neutral gewaschen und im Vakuum bei 40 bis 50 °C bis zur Gewichtskonstanz getrocknet.

Man erhält 165,4 Teile 3-Chlor-4-oxethylmercapto-benzaldehyd vom Schmelzpunkt 77 bis 79 °C und einem Reingehalt von 97,3 % (HPLC). Das Produkt enthält ca. 1 % 3-Chlor-4-oxethylmercapto-benzylalkohol. Durch Ausrühren mit 300 Teilen 60 °C warmem Wasser kann diese Verunreinigung entfernt werden. Dabei resultieren 161,9 Teile 3-Chlor-4-oxethylmercapto-benzaldehyd vom Schmelzpunkt 79 bis 80 °C (Reingehalt durch HPLC: 98,9 %). Wird bei der vorstehend beschriebenen Umsetzung die Menge an Mercaptoethanol und an Kalilauge um 10 % erhöht, verkürzt sich die Reaktionszeit um ca. 2 Stunden. Ausbeute und Qualität bleiben unverändert.

**Beispiel 4**

Zu 1190 Teilen 20%iger Kalilauge tropft man unter Rühren bei 15 biz 20 °C innerhalb 60 Minuten 390 Teile Mercaptoethanol, heizt auf 70 bis 75 °C und gibt dann auf einmal 286 Teile einer 75 bis 80 °C warmen 2,4-Dichlorbenzaldehyd-Schmelze zu.

Man heizt zügig auf 90 bis 95 °C weiter, hält 6 Stunden bei dieser Temperatur und steigert dan auf 110 bis 115 °C (Rückfluß). Man rührt so lange bei Rückfluß nach, bis durch HPLC kein Ausgangsprodukt mehr nachweisbar und ein nahezu einheitliches Reaktionsprodukt (> 95 Flächenprozent) identifizierbar ist, was ca. 13 bis 15 Stunden erfordert. Anschließend setzt man zum Lösen der anorganischen Salze 500 Teile Wasser zu, kühlt unter Rühren auf 0 bis 5 °C ab und isoliert den ausgefallenen Niederschlag durch Filtration.

Man erhält nach Waschen mit 500 Teilen Eiswasser und Trocknen im Vakuum bei Raumtemperatur 335,2 Teile 2,4-Bis-oxethylmercapto-benzaldehyd vom Schmelzpunkt 76 bis 78 °C und einem Reingehalt von 98,1 % (HPLC).
(Analyse: S: 25,0/24,8 %; ber. 24,81 %/Restchlor: < 0,3 %; ber. 0,0 %).

**Beispiele 5 bis 8**

Ersetzt man in den Beispielen 1 bis 4 den jeweils eingesetzten Aldehyd durch aliquote Teile der in Tabelle 1 aufgeführten Aldehyde und arbeitet sonst in der angegebenen Weise, bis chromatographisch der Nachweis auf vollständige Umsetzung erbracht werden kann, so erhält man die aus der nachstehenden Tabelle 1 ersichtlichen Oxethylmercapto-benzaldehyde der allgemeinen Formel II (siehe weiter unten) mit den aufgeführten Reinheiten (HPLC) in den dort niedergelegten Ausbeuten.

Tabelle 1

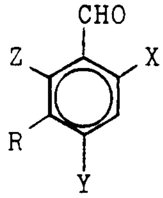

(II)

| Bsp. | Ausgangs-verbindung | Produkt X | Produkt Y | Produkt Z | Produkt R | Ausbeute | RG |
|---|---|---|---|---|---|---|---|
| 5 | 2,5-Dibrom-benzaldehyd | $SC_2H_4OH$ | H | H | Br | 94,4 % | 97,8 % |
| 6 | 2,6-Dichlor-benzaldehyd | $SC_2H_4OH$ | H | $SC_2H_4OH$ | H | 81,0 % | 98,4 % |
| 7 | 2,3-Dichlor-benzaldehyd | H | H | $SC_2H_4OH$ | Cl | 75,8 % | 97,1 % |
| 8 | 2,4,5-Trichlor-benzaldehyd | $SC_2H_4OH$ | $SC_2H_4OH$ | H | Cl | 91,2 % | 97,5 % |

("RG" bedeutet Reingehalt)

**Beispiel 9**

364 Teile 4-Oxethylmercapto-benzaldehyd, 500 Teile Wasser und 6 Teile Natriumwolframat werden bei 60°C verrührt und die sich bildende Emulsion mit 2n Schwefelsäure auf pH 1,5 gestellt. Man tropft innerhalb 2 Stunden 408 Teile 35%iges Wasserstoffperoxid zu, steigert die Innentemperatur anschließend auf 75°C und rührt ca. 14 Stunden nach, bis eine dünnschichtchromatografische Analyse die Abwesenheit von 4-Oxethylmercapto-benzaldehyd anzeigt. Dann kühlt man unter Rühren auf Raumtemperatur, stellt mit 2n Natronlauge einen pH-Wert von 8,5 ein, saugt den suspendierten 4-Oxethylsulfonyl-benzaldehyd ab, wäscht mit Kaltwasser neutral und trocknet im Vakuum bei 50°C.

Man erhält 340 Teile 4-Oxethylsulfonyl-benzaldehyd vom Schmelzpunkt 111 bis 113°C und einem Reingehalt (HPLC) von 97,2 %. Die Verbindung zeigt alle typischen Aldehyd-Reaktionen.

**Beispiel 10**

Ersetzt man im Beispiel 9 den 4-Oxethylmercapto-benzaldehyd durch 2-Oxethylmercapto-benzaldehyd und arbeitet im übrigen in der angegebenen Weise, so erhält man den 2-Oxethylsulfonyl-benzaldehyd in vergleichbarer Ausbeute und Qualität.

**Beispiel 11**

546 Teile 4-Oxethylmercapto-benzaldehyd, 780 Teile Wasser, 15 Teile (R)Arkopal N 100 (Handelsprodukt der HOECHST AG) und 8 Teile Natriumwolframat werden bei 60°C verrührt und die dabei entstehende Emulsion mit 4n Natronlauge auf pH 9 gestellt. Nun tropft man in 60 Minuten 1000 Teile 35%iges Wasserstoffperoxid zu, wobei die Temperatur bis auf 95°C ansteigen darf, und heizt 15 bis 29 Stunden bei 95°C nach, bis eine HPLC-Probe vollständigen Umsatz anzeigt. Anschließend setzt man 500

Teile Wasser zu, kühlt unter Rühren auf Raumtemperatur ab, stellt mit 2n Salzsäure auf pH 6 und saugt den gebildeten Niederschlag ab.

Nach Neutralwaschen mit Eiswasser und Trocknen in Vakuum bei 80 °C erhält man 614 Teile 4-Oxethylsulfonyl-benzoesäure vom Schmelzpunkt 169 bis 172 °C und einem Reingehalt von 95,8 % (HPLC). Eine aus Wasser unter Zusatz von Aktivkohle umkristallisierte Probe zeigt einen Schmelzpunkt von 190 °C und einen Reingehalt von 99,1 % (HPLC).

**Beispiele 12 bis 16**

Ersetzt man den im Beispiel 11 aufgeführten 4-Oxethylmercapto-benzaldehyd durch aliquote Teile der in der nachstehenden Tabelle 2 angegebenen Aldehyde und verfährt im übrigen in analoger Weise, bis chromatografisch der Nachweis auf vollständige Umsetzung erbracht ist, so erhält man die aus Tabelle 2 ersichtlichen Oxethylsulfonylbenzoesäuren der weiter unten aufgeführten allgemeinen Formel (III) mit den dort angegebenen Reingehalten (HPLC) und Ausbeuten.

Tabelle 2

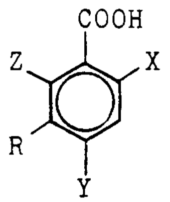

(III)

| Bsp. | Ausgangs-verbindung | Produkt X | Y | Z | R | Ausbeute | RG |
|---|---|---|---|---|---|---|---|
| 12 | 2-Oxethylmercapto-benzaldehyd | $SO_2C_2H_4OH$ | H | H | H | 85,9 % | 96,5 % |
| 13 | 3-Chlor-4-oxethyl-mercapto-benzaldehyd | H | $SO_2C_2H_4OH$ | H | Cl | 92,3 % | 98,1 % |
| 14 | 2,4-Bisoxethyl-mercapto-benzaldehyd | $SO_2C_2H_4OH$ | $SO_2C_2H_4OH$ | H | H | 79,4 % | 94,9 % |
| 15 | 3-Chlor-2-oxethyl-mercapto-benzaldehyd | H | H | $SO_2C_2H_4OH$ | Cl | 93,1 % | 97,5 % |
| 16 | 5-Chlor-2-oxethyl-mercapto-benzaldehyd | $SO_2C_2H_4OH$ | H | H | Cl | 94,0 % | 96,8 % |

**Beispiel 17**

Eine Mischung aus 213,4 Teilen 25%iger Kalilauge und 88,3 Teilen Mercaptoethanol wird unter Rühren innerhalb 30 Minuten bei 35 bis 40 °C mit 114,8 Teilen geschmolzenem 4-Chlorbenzaldehyd versetzt. Nachfolgend steigert man die Temperatur auf 95 °C und rührt 15 Stunden bis zur vollständigen Umsetzung nach (Kontrolle durch Dünnschichtchromatografie). Man verdünnt durch Zugabe von 50 Teilen Wasser. Der pH-Wert der Reaktionsmischung beträgt ca. 9,5 bis 10,0.

Zur Oxidation werden nun 2 Teile Natriumwolframat zugesetzt und innerhalb 90 Minuten bei weiterhin 90 bis 95 °C 380 Teile 30%iges Wasserstoffperoxid zugetropft. Man hält ca. 18 Stunden bei dieser Temperatur, setzt 200 Teile Wasser zu, kühlt auf 50 bis 60 °C ab und stellt mit 2n Salzsäure auf den pH-Wert 6.

Anschließend wird weiter auf 10 bis 15 °C abgekühlt, der angefallene Niederschlag abgesaugt, mit Eiswasser salzfrei gewaschen und im Vakuum bei 80 °C getrocknet. Man erhält 160 Teile 4-Oxethylsulfonyl-benzoesäure vom Schmelzpunkt 168 bis 170 °C und einem Reingehalt von 95,6 % (HPLC).

Wird nach beendeter Oxidation durch Zugabe von ausreichend 2n Natronlauge (ca. 400 Volumenteile) der ausgefallene Niederschlag bei 90 °C in Lösung gebracht, die Lösung mit 10 Teilen Aktivkohle versetzt und anschließend bei 90 °C geklärt und das Filtrat dann mit 2n Salzsäure neutralisiert, so erhält man nach Kühlen, Absaugen, Waschen und Trocknen 148 Teile 4-Oxethylsulfonyl-benzoesäure vom Schmelzpunkt 189 bis 191 °C und einem Reingehalt von 98,9 % (HPLC).

**Beispiele 18 bis 21**

Ersetzt man im Beispiel 17 den 4-Chlorbenzaldehyd durch aliquote Teile der in der nachstehenden Tabelle 3 aufgeführten Halogenbenzaldehyde und arbeitet im übrigen in der angegebenen Weise, so erhält man die aus Tabelle 3 ersichtlichen Oxethylsulfonyl-benzoesäuren der Formel (III) (siehe weiter unten) mit den dort niedergelegten Ausbeuten und Reingehalten (HPLC).

Tabelle 3

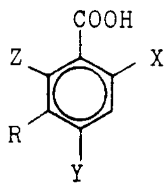

(III)

| Bsp. | Ausgangs-verbindung | Produkt X | Y | Z | R | Direkt isoliert Ausb. | RG | Kohleklärung Ausb. | RG |
|---|---|---|---|---|---|---|---|---|---|
| 18 | 3,4-Dichlor-benzaldehyd | H | $SO_2C_2H_4OH$ | H | Cl | 91,8 % | 96,8 % | 85,3 % | 99,1 % |
| 19 | 2,5-Dibrom-benzaldehyd | $SO_2C_2H_4OH$ | H | H | Br | 94,1 % | 93,4 % | 90,5 % | 98,5 % |
| 20 | 2,3-Dichlor-benzaldehyd | H | H | $SO_2C_2H_4OH$ | Cl | 92,2 % | 92,8 % | 86,4 % | 98,7 % |
| 21 | 2-Fluor-benzaldehyd | $SO_2C_2H_4OH$ | H | H | H | 89,6 % | 95,1 % | 83,8 % | 99,0 % |

**Patentansprüche**

1. Verfahren zur Herstellung von Oxethylmercapto-benzaldehyden und deren Oxidationsprodukten Oxethylsulfonyl-benzaldehyde und Oxethylsulfonyl-benzoesäuren der allgemeinen Formel (I)

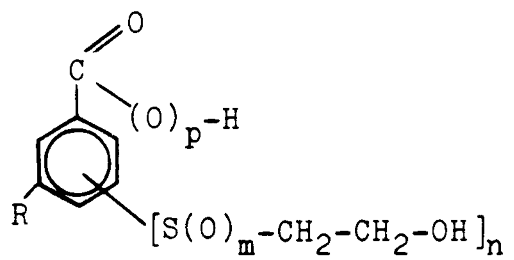

(I)

in welcher R ein Wasserstoff-, Fluor-, Chlor-, Brom-oder Jodatom, m die Zahl 0 oder 2, n die Zahl 1 oder 2, und p die Zahl 0 oder 1 bedeuten, mit der Maßgabe, daß p = 0, wenn m = 0 ist, und die Seitenkette $-S(O)_m-CH_2-CH_2-OH$ in ortho- und/oder para-Position zur Aldehyd- bzw. Carboxygruppe steht, dadurch gekennzeichnet, daß man 1 Mol eines Halogenbenzaldehyds der allgemeinen Formel (II)

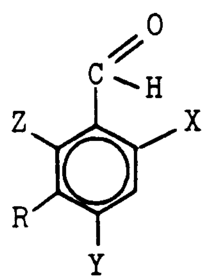

(II)

in welcher R, X, Y und Z Wasserstoff-, Fluor-, Chlor-, Brom- oder Jodatome darstellen, mit der Maßgabe, daß R, X, Y und Z in summa 1, 2 oder 3 Halogenatome sein können, wobei R ein Halogenatom ist, wenn R, X, Y und Z zusammen 3 Halogenatome darstellen, in wäßrigem Medium in Abwesenheit organischer Lösungsmittel mit 1,0 bis 1,5 Mol Mercaptoethanol (pro auszutauschendem Halogenatom) in Gegenwart säurebindender Mittel bei Temperaturen von 70 °C bis 150 °C, zu den entsprechenden Oxethylmercapto-benzaldehyden kondensiert und diese zu den entsprechenden Oxethylsulfonyl-benzaldehyden bzw. Oxethylsulfonyl-benzoesäuren mindestens mit der jeweils erforderlichen Menge Wasserstoffperoxid bei Temperaturen von 40 °C bis 110 °C in Gegenwart einer Wolfram-(VI)-Verbindung als Katalysator oxidiert.

**2.** Verfahren nach Anspruch 1, dadurch gekennzeichnet, daß man zum Erhalt des entsprechenden Oxethylsulfonyl-benzaldehyds bei pH-Werten < 5 oxidiert.

**3.** Verfahren nach Anspruch 1, dadurch gekennzeichnet, daß man zum Erhalt der entsprechenden Oxethylsulfonyl-benzoesäuren bei pH-Werten > 8 oxidiert.

**4.** Verfahren nach mindestens einem der Ansprüche 1 bis 4, dadurch gekennzeichnet, daß man die Kondensation in Gegenwart von Ätznatron, Natronlauge, Ätzkali, Kalilauge oder Magnesiumoxid durchführt.

**5.** Verfahren nach mindestens einem der Ansprüche 1 bis 4, dadurch gekennzeichnet, daß man die Kondensation bei Temperaturen von 90 °C bis 120 °C durchführt.

**6.** Verfahren nach mindestens einem der Ansprüche 1 bis 5, dadurch gekennzeichnet, daß man 1 Mol der Ausgangsverbindung der dort genannten Formel (II) mit 1,25 bis 1,4 Mol Mercaptoethanol kondensiert.

**7.** Verfahren nach mindestens einem der Ansprüche 1 bis 6, dadurch gekennzeichnet, daß man die bei der Kondensation erhaltenen Oxethylmercapto-benzaldehyde (zum Erhalt der entsprechenden Oxethylsulfonyl-benzaldehyde) mit mindestens 2 Mol Wasserstoffperoxid pro Oxethylmercaptogruppe oxidiert.

**8.** Verfahren nach mindestens einem der Ansprüche 1 bis 6, dadurch gekennzeichnet, daß man bei der Kondensation erhaltene Mono-Oxethylmercapto-benzaldehyde (zum Erhalt der entsprechenden Mono-Oxethylsulfonyl-benzoesäuren) mit mindestens 3 Mol Wasserstoffperoxid oxidiert.

**9.** Verfahren nach mindestens einem der Ansprüche 1 bis 6, dadurch gekennzeichnet, daß man bei der Kondensation erhaltene Di-Oxethylmercapto-benzaldehyde (zum Erhalt der entsprechenden Di-Oxethylsulfonyl-benzoesäuren) mit mindestens 5 Mol Wasserstoffperoxid oxidiert.

**10.** Verfahren nach mindestens einem der Ansprüche 1 bis 9, dadurch gekennzeichnet, daß man die Oxidation bei Temperaturen von 60 °C bis 95 °C durchführt.

**11.** Verfahren nach mindestens einem der Ansprüche 1 bis 10, dadurch gekennzeichnet, daß man die Oxidation der zunächst erhaltenen Oxethylmercaptobenzaldehyde zu den entsprechenden Oxethylsulfonyl-benzaldehyden bei pH-Werten zwischen 1 und 4, und zu den entsprechenden Oxethylsulfonyl-benzoesäuren bei pH-Werten zwischen 9 und 13 vornimmt.

**12.** Verfahren nach mindestens einem der Ansprüche 1 bis 11, dadurch gekennzeichnet, daß man die Oxidation in Gegenwart von Natriumwolframat oder Wolframtrioxid als Katalysator vornimmt.

## Claims

**1.** A process for the preparation of oxethylmercaptobenzaldehydes and their oxidation products oxethylsulfonylbenzaldehydes and oxethylsulfonylbenzoic acids of the formula (I)

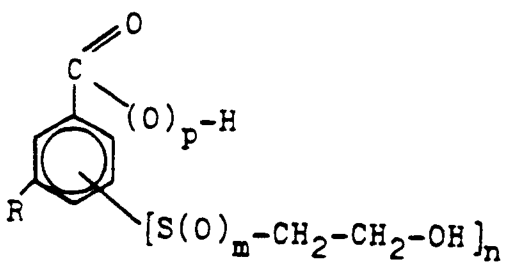

(I)

in which R denotes a hydrogen, fluorine, chlorine, bromine or iodine atom, m denotes the number 0 or 2, n denotes the number 1 or 2, and p denotes the number 0 or 1, with the proviso that p is 0 if m is 0, and the side-chain $-S(O)_m-CH_2-CH_2-OH$ is in the ortho and/or para position to the aldehyde or carboxyl group, which comprises condensing 1 mole of a halobenzaldehyde of the formula (II)

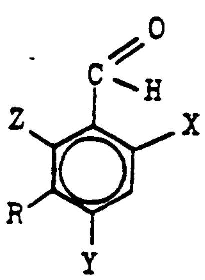

(II)

in which R, X, Y and Z represent hydrogen, fluorine, chlorine, bromine or iodine atoms, with the proviso that R, X, Y and Z can be in total 1, 2 or 3 halogen atoms, where R is a halogen atom if R, X, Y and Z together represent 3 halogen atoms, in aqueous medium in the absence of organic solvents with 1.0 to 1.5 moles of mercaptoethanol (per halogen atom to be exchanged) in the presence of acid-binding agents at temperatures of 70 °C to 150 °C, to give the corresponding oxethylmercaptobenzaldehydes and oxidizing these to give the corresponding oxethylsulfonylbenzoic acids, or oxethylsulfonylbenzoic acids using at least the amount of hydrogen peroxide necessary in each case at temperatures of 40 °C to 110 °C in the presence of a tungsten (VI) compound as a catalyst.

2. The process as claimed in claim 1, wherein, to obtain the corresponding oxethylsulfonylbenzaldehyde, the oxidation is carried out at pH < 5.

3. The process as claimed in claim 1, wherein, to obtain the corresponding oxethylsulfonylbenzoic acids, the oxidation is carried out at pH > 8.

4. The process as claimed in at least one of claims 1 to 4, wherein the condensation is carried out in the presence of caustic soda, sodium hydroxide solution, caustic potash, potassium hydroxide solution or magnesium oxide.

5. The process as claimed in at least one of claims 1 to 4, wherein the condensation is carried out at temperatures of 90 ° C to 120 ° C.

6. The process as claimed in at least one of claims 1 to 5, wherein 1 mole of the starting compound of the formula (II) mentioned therein is condensed with 1.25 to 1.4 moles of mercaptoethanol.

7. The process as claimed in at least one of claims 1 to 6, wherein the oxethylmercaptobenzaldehydes obtained in the condensation (to obtain the corresponding oxethylsulfonylbenzaldehydes) are oxidized with at least 2 moles of hydrogen peroxide per oxethylmercapto group.

8. The process as claimed in at least one of claims 1 to 6, wherein mono-oxethylmercaptobenzaldehydes obtained in the condensation (to obtain the corresponding mono-oxethylsulfonylbenzoic acids) are oxidized with at least 3 moles of hydrogen peroxide.

9. The process as claimed in at least one of claims 1 to 6, wherein di-oxethylmercaptobenzaldehydes obtained in the condensation (to obtain the corresponding di-oxethylsulfonylbenzoic acids) are oxidized with at least 5 moles of hydrogen peroxide

10. The process as claimed in at least one of claims 1 to 9, wherein the oxidation is carried out at temperatures of 60 ° C to 95 ° C.

11. The process as claimed in at least one of claims 1 to 10, wherein the oxidation of the oxethylmercaptobenzaldehydes first obtained to give the corresponding oxethylsulfonylbenzaldehydes is performed at a pH between 1 and 4, and wherein said oxidation to give the corresponding oxethylsulfonylbenzoic acids is performed at a pH between 9 and 13.

12. The process as claimed in at least one of claims 1 to 11, wherein the oxidation is performed in the presence of sodium tungstate or tungsten trioxide as a catalyst.

## Revendications

1. Procédé pour préparer des hydroxyéthylthiobenzaldéhydes et les produits d'oxydation de ceux-ci que sont les hydroxyéthylsulfonyl-benzaldéhydes et les acides hydroxyéthylsulfonyl-benzoïques, qui répondent à la formule générale I :

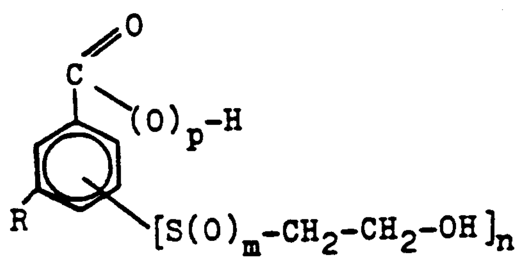

(I)

dans laquelle R représente un atome d'hydrogène, de fluor, de chlore, de brome ou d'iode, m est égal à 0 ou à 2, n est égal à 1 ou à 2, et p est égal à 0 ou à 1, avec la condition que p soit égal à 0 lorsque m est égal à 0, et la chaîne latérale $-S(O)_m-CH_2-CH_2-OH$ est en position ortho et/ou para relativement

au radical formyle ou carboxy, caractérisé en ce qu'on condense 1 mol d'un halogénobenzaldéhyde repondant à la formule générale II :

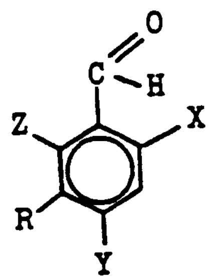

(II)

dans laquelle R, X, Y et Z représentent chacun un atome d'hydrogène, de fluor, de chlore, de brome ou d'iode, avec la condition que R, X, Y et Z puissent représenter au total 1, 2 ou 3 atomes d'halogène, le symbole R représentant un atome d'halogène lorsque R, X, Y et Z représentent ensemble trois atomes d'halogène,
en milieu aqueux (en l'absence de solvants organiques), avec de 1,0 à 1,5 mol de mercapto-éthanol (par atome d'halogène à échanger), en présence d'accepteurs d'acides, à des températures de 70 à 150 °C, condensation qui conduit aux hydroxyéthylthio-benzaldéhydes correspondants, et on oxyde ceux-ci en les hydroxyéthylsulfonyl-benzaldéhydes ou les acides hydroxyéthylsulfonyl-benzoïques correspondants avec au moins la quantité nécessaire à chaque fois de peroxyde d'hydrogène, à des températures de 40 à 110 °C, en présence d'un composé du tungstène (VI) comme catalyseur.

**2.** Procédé selon la revendication 1 caractérisé en ce que, pour obtenir l'hydroxyéthylsulfonyl-benzaldéhyde correspondant, on oxyde à des pH inférieurs à 5.

**3.** Procédé selon la revendication 1 caractérisé en ce que, pour obtenir les acides hydroxyéthylsulfonyl-benzoïques correspondants, on oxyde à des pH supérieurs à 8.

**4.** Procédé selon l'une quelconque des revendications de 1 à 4, caractérisé en ce qu'on effectue la condensation en présence d'hydroxyde de sodium, d'une solution d'hydroxyde de sodium, d'hydroxyde de potassium, d'une solution d'hydroxyde de potassium, ou d'oxyde de magnésium.

**5.** Procédé selon l'une quelconque des revendications 1 à 4, caractérisé en ce qu'on effectue la condensation à des températures de 90 à 120 °C.

**6.** Procédé selon l'une quelconque des revendications 1 à 5, caractérisé en ce qu'on condense 1 mol du composé de départ répondant à la formule II mentionnée, avec de 1,25 à 1,4 mol de mercapto-éthanol.

**7.** Procédé selon l'une quelconque des revendications 1 à 6, caractérisé en ce qu'on oxyde les hydroxyéthylthio-benzaldéhydes obtenus lors de la condensation (pour obtenir les hydroxyéthylsulfonyl-benzaldéhydes correspondants) avec au moins 2 mol de peroxyde d'hydrogène par radical hydroxyéthylthio.

**8.** Procédé selon l'une quelconque des revendications 1 à 6, caractérisé en ce qu'on oxyde des mono-(hydroxyéthylthio)-benzaldéhydes obtenus lors de la condensation [pour obtenir les acides mono-(hydroxyéthylsulfonyl)-benzoïques correspondants], avec au moins 3 mol de peroxyde d'hydrogène.

**9.** Procédé selon l'une quelconque des revendications 1 à 6, caractérisé en ce qu'on oxyde des bis-(hydroxyéthylthio)-benzaldéhydes obtenus lors de la condensation [pour obtenir les acides bis-(hydroxyéthylsulfonyl)-benzoïques correspondants], avec au moins 5 mol de peroxyde d'hydrogène.

**10.** Procédé selon l'une quelconque des revendications 1 à 9, caractérisé en ce qu'on effectue l'oxydation à des températures de 60 à 95 °C.

**11.** Procédé selon l'une quelconque des revendications 1 à 10, caractérisé en ce qu'on effectue l'oxydation

des hydroxyéthylthio-benzaldéhydes tout d'abord obtenus, pour les convertir en les hydroxyéthylsulfonylbenzaldéhydes correspondants, à des pH compris entre 1 et 4, et, pour les convertir en les acides hydroxyéthylsulfonyl-benzoïques correspondants, à des pH compris entre 9 et 13.

**12.** Procédé selon l'une quelconque des revendications 1 à 11, caractérisé en ce qu'on effectue l'oxydation en présence de tungstate de sodium ou de trioxyde de tungstène comme catalyseur.